# EUROPEAN PATENT APPLICATION

(11) **EP 4 702 918 A1**
(43) Date of publication of application: **04.03.2026**
(21) Application number: 24196352.9
(22) Date of filing: 26.08.2024
(51) Int. Cl.: A61B 5/024, A61B 5/1455, A61B 5/00

(54) **METHOD AND SYSTEM FOR CONTROLLING AN OPTICAL VITAL SIGNS SENSING DEVICE**

(71) Applicant: Koninklijke Philips N.V., 5656 AG Eindhoven (NL)
(72) Inventor: JOOSTEN, Franciscus Ivo, Eindhoven (NL); LUO, Ningqi, Eindhoven (NL)
(74) Representative: Philips Intellectual Property & Standards

(57) **Abstract**

Provided is a method (100) for controlling an optical vital sign sensing device (10). The method includes activating (106) a transmission mode of the optical vital signs sensing device (10) if movement of the optical vital sign sensing device (10) satisfies a movement criterion and/or if a reflection mode signal quality, e.g. signal strength, of the optical vital sign sensing device (10) satisfies a reflection mode minimum quality criterion. Further provided is a processor (28) for controlling the optical vital signs sensing device (10) and an optical vital signs sensing device (10) comprising the processor (28).

## Description

### FIELD OF THE INVENTION

The invention relates to a method, and related computer program, for controlling an optical vital signs sensing device.

The invention further relates to a processor for controlling such an optical vital signs sensing device, and an optical vital signs sensing device comprising said processor.

### BACKGROUND OF THE INVENTION

Optical vital sign sensing devices, such as plethysmography (PPG) sensing devices, are known which are capable of performing vital signs measurements when arranged on a body part. Such devices may be capable of operating in either a transmission mode or a reflection mode. In some cases, the device may be capable of operating in both the transmission mode and the reflection mode, e.g. a dual mode optical vital signs sensing device.

In the transmission mode, an emitter and a detector of the optical vital signs sensing device are separated from each other by the body part such that light is transmitted from the detector at one side of the body part, through the body part, before being detected by the detector on an opposing side of the body part. For example, the emitter and the detector may be positioned on opposing sides of a finger clip.

In contrast, in the reflection mode, the emitter and detector may be positioned on the same side of the body part such that the detector receives light which has been emitted into and reflected back from the body part. For example, the emitter and detector may be positioned adjacent to each other on the underside of a watch with PPG sensing capability.

The reflective measurement principle can be advantageous in terms of cost and ease of manufacturing of the device, since the emitter and detector can be arranged side by side and integrated into the same circuit board and housing. However, whilst the reflective principle may work well for acquiring heart rate measurements, it may be less effective at acquiring measurements such as blood oxygen (SpO2) and breathing rate.

Devices which are capable of performing measurements in the transmission mode may be more complex in terms of manufacturing, e.g. due to positioning of the emitter and detector on opposing sides of the device. However, transmission measurements may enable more detailed information to be gathered, as compared to reflective measurements. For example, it may be possible to obtain SpO2 and breathing rate measurements, as well as heart rate measurements, when performing measurements in the transmission mode.

### SUMMARY OF THE INVENTION

Depending on certain conditions, the reliability of measurements performed in the transmission mode and the reflectance mode may vary. One potential approach to ensuring reliability of results is to provide a user of the device with manual control options, such as the ability to selectively activate or deactivate either mode. However, the effectiveness of such an approach can be dependent upon the user's skill and experience.

More generally, it would be desirable to provide a method of controlling an optical vital signs sensing device, to improve the likelihood of performing reliable vital signs measurements.

The invention is defined by the claims.

According to examples in accordance with a first aspect, there is provided a method comprising: receiving at least one of motion data and reflection mode data, wherein the motion data is indicative of movement of an optical vital signs sensing device and wherein the reflection mode data is associated with a reflection mode signal quality of a vital signs measurement performed by the optical vital signs sensing device in a reflection mode; determining at least one of whether the movement satisfies a movement criterion by analyzing the motion data; and whether the reflection mode signal quality satisfies a reflection mode minimum quality criterion by analyzing the reflection mode data; and activating a transmission mode of the optical vital signs sensing device if at least one of the movement criterion and the reflection mode minimum quality criterion is satisfied.

By activating the transmission mode in this way, the likelihood of obtaining reliable vital signs measurements using the optical vital signs sensing device may be increased. This is because the transmission mode will only be activated when data, e.g. motion data and/or reflection mode data, indicates that reliable transmission mode vital signs measurements will be obtained following activation of the transmission mode.

For example, if vital signs measurements performed in the reflection mode are good quality, e.g. the reflection mode signal quality satisfies the minimum quality criterion, this may indicate a greater likelihood of obtaining reliable measurements in the transmission mode. Similarly, if movement of the device is sufficiently low, e.g. the movement of the device satisfies the movement criterion, this may indicate a greater likelihood of obtaining reliable measurements in the transmission mode, due to the greater sensitivity of transmission measurements to motion.

The optical vital signs sensing device may comprise an optical emitter assembly and an optical detector assembly, the device being controllable to perform vital signs measurements in a transmission mode in which light from the optical emitter assembly is transmitted through the body part to reach the optical detector assembly, e.g. a first optical detector of the optical detector assembly. In other words, the device may be controllable to selectively activate or deactivate the transmission mode.

Additionally, the optical vital signs sensing device may be controllable to perform vital signs measurements, when the device is arranged on a body part, in a reflection mode in which light from the optical emitter assembly is emitted into and reflected from the body part to the optical detector assembly, e.g. a second optical detector of the optical detector assembly. That is, the optical vital signs sensing device may be controllable to perform vital signs measurements in the reflection mode and the transmission mode such that measurements may be performed in either mode individually, or in both modes simultaneously. In other words, the device may be controllable to selectively activate or deactivate the transmission mode and the reflection mode. Such a device may be termed a dual-mode optical vital signs sensing device.

The motion data may comprise accelerometer data indicative of acceleration of the optical vital signs sensing device. In such embodiments, the movement criteria may be satisfied when the motion data, e.g. the accelerometer data, is indicative of movement, e.g. acceleration, of the device being below an acceleration threshold.

The reflection mode signal quality may comprise one or more of a signal strength and/or a signal to noise ratio of the vital signs measurement performed by the optical vital signs sensing device in the reflection mode.

In such embodiments, the reflection mode minimum quality criterion may be satisfied when the signal strength is above a minimum signal strength and/or when the signal to noise ratio is above a minimum signal to noise ratio.

In embodiments in which the optical vital signs sensing device is controllable to perform vital signs measurements, when the device is arranged on a body part, in a reflection mode, the motion data may be indicative of movement of the optical vital sign sensing device whilst the reflection mode is active.

The method may be a processor-implemented method. For example, the receiving at least one of motion data and reflection mode data may be by an input port of a processor, and data processed by the processor may be outputted by another port of the processor, e.g. an output port.

In some embodiments, the receiving comprises receiving the motion data and the reflection mode data, the determining comprises determining whether the movement satisfies the movement criterion and whether the reflection mode signal quality satisfies the reflection mode minimum signal criterion, and the activating the transmission mode is subject to the movement criterion and the reflection mode minimum quality criterion being satisfied.

In this way, the likelihood of obtaining reliable transmission mode vital signs measurements may be increased, compared to the situation when only one of the movement criterion or the reflection mode minimum signal quality criterion is satisfied.

In some embodiments, the method further comprises receiving transmission mode data, wherein the transmission mode data is associated with a transmission mode signal quality of a vital signs measurement performed by the optical vital signs sensing device in the transmission mode.

That is, the transmission mode data may be received following the activating of the transmission mode.

In this way, the optical vital signs sensing device may be further controlled whilst the transmission mode is active.

In embodiments in which the optical vital signs sensing device is a dual-mode optical vital signs sensing device, the transmission mode data may be received whilst both the reflection mode and the transmission mode are active, or whilst the transmission mode is active, and the reflection mode is inactive.

In some embodiments, the method further comprises determining whether the transmission mode signal quality satisfies a transmission mode minimum quality criterion by analyzing the transmission mode data, and deactivating the reflection mode if the transmission mode minimum quality criterion is satisfied.

In this way, the likelihood of obtaining reliable measurements may be increased. This is because the reflection mode may not be deactivated until reliable measurements have been obtained in the transmission mode. This may ensure continuity of measurement.

The transmission mode signal quality may comprise one or more of a signal strength and/or a signal to noise ratio of a vital signs measurement performed by the optical vital signs sensing device in the transmission mode. That is, the transmission mode minimum quality criterion may be satisfied when the signal strength is above a minimum signal strength and/or when the signal to noise ratio is above a minimum signal to noise ratio.

In some embodiments, the method further comprises determining whether movement of the optical vital signs sensing device while the transmission mode is active satisfies a further movement criterion by analyzing transmission mode motion data, and wherein the method further comprises at least one of deactivating the transmission mode if the further movement criterion is satisfied and activating the reflection mode if the further movement criterion is satisfied.

In this way, the likelihood of obtaining reliable measurements may be increased, whilst also ensuring continuity of measurement.

The transmission mode motion data may comprise transmission mode accelerometer data indicative of acceleration of the optical vital signs sensing device whilst the transmission mode is active. In such embodiments, the further movement criterion may be satisfied when the transmission mode motion data, e.g. the transmission mode accelerometer data, is indicative of movement, e.g. acceleration, of the device being above a transmission mode acceleration threshold.

In some embodiments, the method further comprises determining whether movement of the optical vital signs sensing device while no transmission mode and no reflection mode are active satisfies an initial movement criterion by analyzing initial motion data and activating at least one of the reflection mode and the transmission mode if the initial movement criterion is satisfied.

In this way, the vital signs sensing device may be further controlled whilst the device is not being used to perform vital signs measurements, e.g. whilst the device is in a standby mode, prior to performing a measurement.

The initial motion data may comprise initial accelerometer data indicative of acceleration of the optical vital signs sensing device while no transmission mode and no reflection mode are active. In such embodiments, the initial movement criterion may be satisfied when the initial motion data, e.g. the initial accelerometer data, is indicative of movement, e.g. acceleration, of the device being above an initial acceleration threshold.

In some embodiments, the method further comprises generating a signal for creating a notification that the vital signs sensing device is being used incorrectly.

In this way, a user of the device may be alerted when the device is being used incorrectly, increasing the likelihood of obtaining reliable vital signs measurements.

In some embodiments, the method further comprises receiving environmental light data of an environment in which the vital signs sensing device is being used, and the generating the signal for creating the notification is based at least partly on the environmental light data.

The environmental light data may comprise a light intensity of light in the environment in which the vital signs sensing device is being used.

Environmental light levels may be indicative of incorrect use, e.g. incorrect placement, of the optical vital signs sensor.

In some embodiments, the generating the signal for creating the notification is based at least partly on the reflection mode data.

Reflection mode data may be indicative of incorrect use of the optical vital signs sensing device. In this way, the likelihood of obtaining reliable optical vital signs measurements may be increased.

For example, low quality vital signs measurements performed in the reflection mode may be indicative of incorrect use of the optical vital signs sensing device.

Alternatively or additionally, the generating the signal for creating the notification may be based on the motion data.

Alternatively or additionally, the generating the signal for creating the notification may be based on the transmission mode motion data and/or the transmission mode data.

The reflection mode data and/or the transmission mode data may be indicative of one or more heart rate measurements, blood oxygen measurements and/or breathing measurements.

For example, the reflection mode data may be indicative of one or more heart rate measurements performed by the device in the reflection mode.

For example, the transmission mode data may be indicative of one or more heart rate measurements, blood oxygen measurements and/or breathing measurements performed by the device in the transmission mode.

In some embodiments, green light is emitted by the emitter assembly whilst the reflection mode is active, and at least one of red and infrared light is emitted by the emitter assembly whilst the transmission mode is active.

According to a second aspect there is provided a computer program comprising computer-readable instructions encoding the steps of the method of any of the embodiments described herein.

In other words, there may be provided a computer program comprising instructions which, when the instructions are executed by a processor, cause the processor to carry out the method according to any of the embodiments described herein.

According to a third aspect there is provided a processor configured to execute the computer program.

In other words, the processor is configured to: receive at least one of: motion data and reflection mode data, wherein the motion data is indicative of movement of an optical vital signs sensing device and wherein the reflection mode data is associated with a reflection mode signal quality of a vital signs measurement performed by the optical vital signs sensing device in a reflection mode; determine at least one of whether the movement satisfies a movement criterion by analyzing the motion data; and whether the reflection mode signal quality satisfies a reflection mode minimum quality criterion by analyzing the reflection mode data; and activate a transmission mode of the optical vital signs sensing device if at least one of the movement criterion and the reflection mode minimum quality criterion is satisfied.

The method may be a processor-implemented method. For example, the receiving at least one of motion data and reflection mode data may be by an input port of a processor, and data processed by the processor may be outputted by another port of the processor, e.g. an output port.

The processor may, for example, be a processor circuit.

According to a fourth aspect there is provided an optical vital signs sensing device comprising: an optical emitter assembly; and an optical detector assembly comprising a first optical detector; the optical vital signs sensing device being configured to perform vital signs measurements, when the device is arranged on a body part, in at least a transmission mode in which light from the optical emitter assembly is transmitted through the body part to reach the first optical detector, and a processor configured to: receive at least one of: motion data and reflection mode data, wherein the motion data is indicative of movement of an optical vital signs sensing device and wherein the reflection mode data is associated with a reflection mode signal quality of a vital signs measurement performed by the optical vital signs sensing device in a reflection mode; determine at least one of: whether the movement satisfies a movement criterion by analyzing the motion data; and whether the reflection mode signal quality satisfies a reflection mode minimum quality criterion by analyzing the reflection mode data; and activate the transmission mode of the optical vital signs sensing device if at least one of the movement criterion and the reflection mode minimum quality criterion is satisfied.

In some embodiments, the optical detector assembly comprises a second optical detector, wherein the optical vital signs sensing device is configured to perform vital signs measurements on the body part in the reflection mode in which light from the optical emitter assembly is emitted into and reflected from the body part to the second optical detector.

In some embodiments the optical vital signs sensing device further comprises a motion sensor for providing the motion data.

The motion sensor may be an accelerometer, and the motion data may be accelerometer data.

According to a fifth aspect there is provided a method comprising causing, using a first processor, a second processor to execute a computer program comprising computer-readable instructions encoding the steps of the method of any of the embodiments described herein.

For example, the second processor may be the processor comprised by the optical vital signs sensing device. The first processor and the second processor may be separate devices that are each included in a processing system, e.g. a distributed system in which the first processor is remote from the second processor.

According to a sixth aspect there is provided a computer program comprising computer-readable instructions encoding the step of the fifth aspect.

In other words, there is provided a computer program comprising instructions which, when the program is executed by a processor, e.g. the first processor, cause another processor, e.g. the second processor, to carry out the step of the method of the fifth aspect.

Further provided is a processor, e.g. the first processor, configured to execute a computer program comprising computer-readable instructions encoding the step of the fifth aspect.

One or more non-transitory computer readable media may be provided, which non-transitory computer readable media have a computer program stored thereon, with the computer program comprising computer program code, which is configured, when the computer program is run on a processor, to cause the processor to implement the method according to any of the embodiments described herein.

More generally, embodiments described herein in relation to the optical vital signs sensing device may be applicable to the method and computer program, and embodiments described herein in relation to the method and computer program may be applicable to the beverage machine.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a block diagram of an optical vital signs sensing device according to an example.
FIG. 2 provides a diagram of a transmissive optical vital signs sensing device according to an example.
FIG. 3 provides an example of a reflective optical vital signs sensing device.
FIG. 4 provides an example of a reflective and transmissive vital signs sensing device according to an example.
FIG. 5 provides a flow chart of a typical use case of an optical vital signs sensing device.
FIG. 6 provides a flow chart of a method according to an example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a method for controlling an optical vital sign sensing device. The method includes activating a transmission mode of the optical vital signs sensing device if movement of the optical vital sign sensing device satisfies a movement criterion and/or if a reflection mode signal quality, e.g. signal strength, of the optical vital sign sensing device satisfies a reflection mode minimum quality criterion.

Further provided is a processor for controlling the optical vital signs sensing device, and an optical vital signs sensing device comprising the processor.

The optical vital signs sensing device, e.g. PPG sensing device, is configured to perform vital signs measurements when the device is arranged on a body part. The optical vital signs sensing device comprises an optical emitter assembly and an optical detector assembly comprising a first optical detector, with the optical vital signs sensing device being configured to perform the vital signs measurements in at least the transmission mode in which light from the optical emitter assembly is transmitted through the body part to reach the first optical detector.

The optical detector assembly may comprise a second optical detector in addition to the first optical detector. When the optical detector assembly comprises a second optical detector, the optical vital signs sensing device may be configured to perform vital signs measurements on the body part in a reflection mode in which light from the optical emitter assembly is emitted into and reflected from the body part to the second optical detector.

The optical vital signs sensing device further comprises a processor for controlling the optical vital sign sensing device, the processor being configured to receive at least one of motion data and reflection mode data, wherein the motion data is indicative of movement of the optical vital signs sensing device and wherein the reflection mode data is associated with a reflection mode signal quality of an optical vital signs sensing device. The processor is further configured to determine at least one of whether the movement satisfies a movement criterion by analyzing the motion data and whether the reflection mode signal quality satisfies a reflection mode minimum quality criterion by analyzing the reflection mode data. The processor is further configured to activate the transmission mode of the optical vital signs sensing device if at least one of the movement criterion and the reflection mode minimum quality criterion is satisfied.

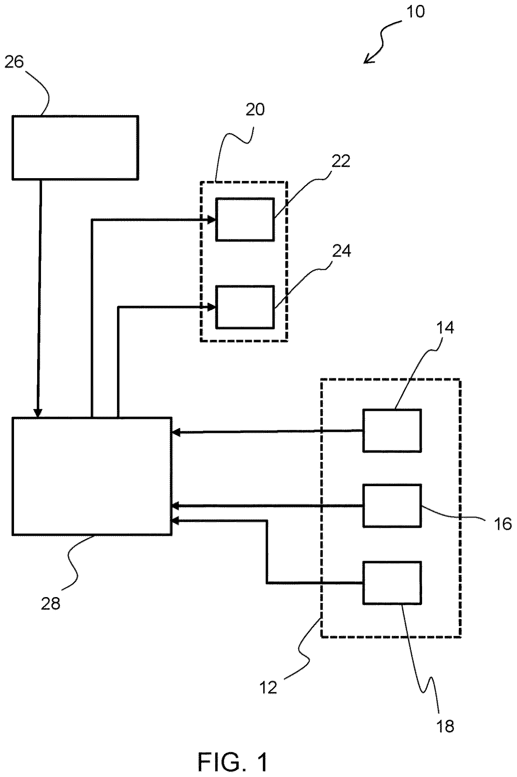

FIG. 1 provides a block diagram of an optical vital signs sensing device 10 according to an example. The optical vital signs sensing device 10 includes an optical emitter assembly 12 for emitting light whilst the device is performing vital signs measurements. To this end the optical emitter assembly 12 may comprise one or more diodes 14, 16, 18.

In some embodiments, such as that shown in FIG. 1, the optical emitter assembly 12 comprises more than one diode, such as diodes 14, 16, 18, which are configured to emit light such as visible light or infrared light whilst performing vital signs measurements. In such embodiments, each diode may be configured to predominantly emit light of a particular wavelength or range of wavelengths. For example, the diode 14 may be configured to emit green light, the diode 16 may be configured to emit red light, and the diode 18 may be configured to emit infrared light.

More generally, the optical emitter assembly 12 may comprise any number of diodes which may be configured to emit light of any wavelength within the visible or infrared spectrum.

In some embodiments, such as that shown in FIG. 1, the optical vital signs sensing device 10 comprises an optical detector assembly 20. The optical detector assembly 20 may comprise a first optical detector 22 configured to receive light emitted by the emitter assembly 12. For example, the first optical detector 22 may comprise one or more photodiodes.

In such embodiments, the first optical detector 22 may be arranged relative to the optical emitter assembly 12, e.g. relative to one or more diodes of the optical emitter assembly 12, such that the optical vital signs sensing device 10 may be configured to perform vital signs measurements in the transmission mode. That is, the optical emitter assembly 12 and the first optical detector 22 may be arranged such that light from the optical emitter assembly 12 is transmitted through the body part to reach the first optical detector 22 whilst performing vital signs measurements.

In some embodiments, such as that shown in FIG. 1, the optical detector assembly 20 may comprise a second optical detector 24 configured to receive light emitted by the emitter assembly 12. For example, the second optical detector 24 may comprise one or more photodiodes.

In such embodiments, the second optical detector 24 may be arranged relative to the optical emitter assembly 12, e.g. relative to one or more diodes of the optical emitter assembly 12, such that the optical vital signs sensing device 10 may be configured to perform vital signs measurements in the reflection mode. That is, the optical emitter assembly 12 and the second optical detector 24 may be arranged such that when measurements are performed on a body part, light from the optical detector assembly 20 is emitted into and reflected from the body part to reach the optical detector 20.

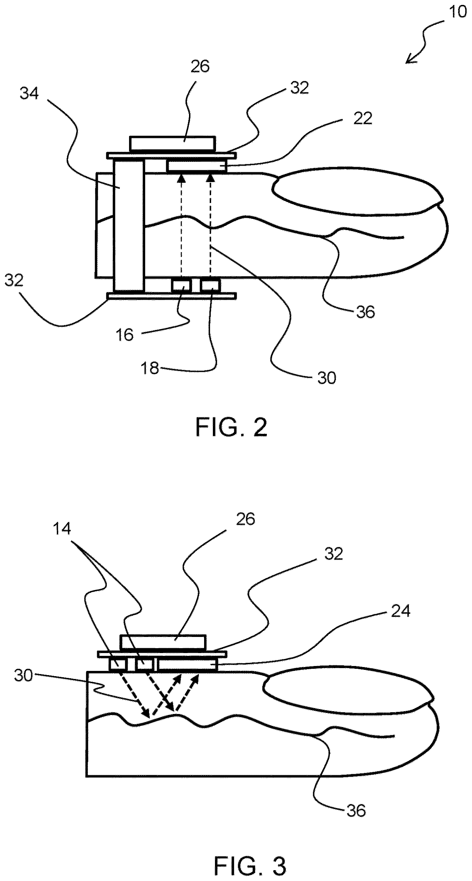

Whilst the optical vital signs sensing device 10 of FIG. 1 is configured to perform measurements in the reflection mode as well as the transmission mode, the optical vital signs sensing device 10 need not be configured to perform measurements in the reflection mode. That is, the device 10 of FIG. 1 may equally be configured such that measurements may be performed in the transmission mode only, such as the optical vital signs sensing device 10 of FIG. 2 discussed below.

FIG. 2 provides a non-limiting illustrative example of an optical vital signs sensing device 10 configured to perform vital signs measurements on a finger in a transmission mode only, e.g. not in the reflection mode. In such an example, diodes 16, 18 may be arranged on an opposing side of the device 10 to the first optical detector 22 comprised by the optical detector assembly 20 (not shown) such that light 30, e.g. red or infrared light, emitted from the diodes 16, 18 passes through the finger, e.g. through a blood vessel 36, to reach the first optical detector 22 when the device 10 is placed on the finger.

FIG. 3 provides a non-limiting illustrative example of an optical vital signs sensing device configured to perform vital signs measurements on a finger in a reflection mode only. In such an example, diode(s) may be arranged on the same side of the device to the second optical detector 24 comprised by the optical detector assembly 20 (not shown) such that light, e.g. green light, is emitted from diode(s) into, and reflected from, the finger, e.g. a blood vessel 36, to the second optical detector 24 when the device is placed on the finger.

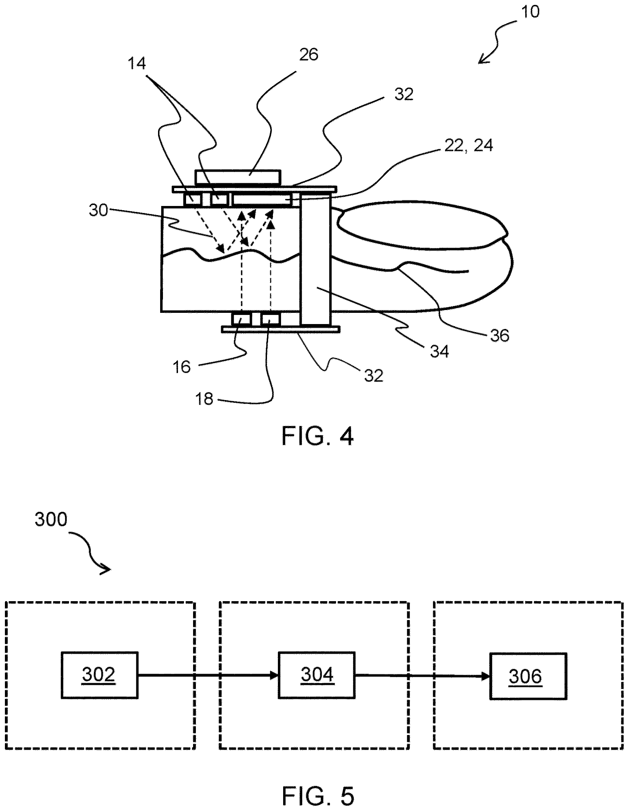

FIG. 4 provides an example of an optical vital signs sensing device 10 configured to perform vital signs measurements on a finger in both the reflection mode and the transmission mode, such as that of FIG. 1. In such an example, diodes 16 and 18 may be arranged on an opposing side of the device 10 to first optical detector 22 comprised by the optical detector assembly 20 (not shown) such that light 30, e.g. red or infrared light, emitted from the diodes 16, 18 passes through the finger, e.g. through a blood vessel 36, to reach the first optical detector 22, as in FIG. 2. In such an example the diode(s) 14 may be arranged on the same side of the device 10 as the second optical detector 24 comprised by the optical detector assembly 20 (not shown) such that light 30, e.g. green light, is emitted into and reflected from the finger, e.g. a blood vessel 36, to the second optical detector 24 as in FIG. 3. In other words, the optical vital signs sensing device 10 of FIG. 4 combines the reflection and transmission sensing capabilities of the assemblies of FIG. 2 and FIG. 3.

In examples of the device 10 which are configured to perform measurements in the transmission mode and the reflection mode, such as those of FIG. 1 and FIG. 4, the first optical detector 22 and the second optical detector 24 may be arranged on the same side of the optical vital signs sensing device 10. Alternatively, the first optical detector 22 and the second optical detector 24 may be arranged on opposing sides of the optical vital signs sensing device 10.

In some embodiments, the optical emitter assembly 12 and the optical detector assembly 20 may be comprised by, e.g. integrated into, one or more circuit boards 32. The one or more circuit boards 32 may be connected via a connector 34.

More generally, the optical vital signs sensing device 10 may comprise a housing and/or securing components (not shown) to enable the device 10 to be secured to the body part whilst performing vital signs measurements in the transmission and/or reflection mode. For example, the device 10 of FIG. 4 may comprise a finger clip (not shown) to enable the device 10 to be secured to the finger whilst performing optical vital signs measurements. It is noted that whilst the illustrative examples of FIGs. 2 to 4 concern an optical vital signs sensing device 10 for performing measurements on a finger, the device 10 may be configured in any suitable way to enable vital signs measurements to be performed on a different body part. For example, the housing and/or securing components may be incorporated into a wrist strap to enable measurements to be performed on a wrist, e.g. the strap of a watch.

Whilst performing reflection mode measurements the emitter assembly 12 may be controlled such that light, e.g. green light, is emitted from one or more diodes, e.g. diode(s) 14, and received by the second optical detector 24. Whilst such reflection mode measurements are being performed, the reflection mode may be considered as being active.

Whilst performing transmission mode measurements the emitter assembly 12 may be controlled such that light, e.g. red and/or infrared light is emitted from one or more diodes, e.g. diodes 16, 18, and received by the first optical detector 22. Whilst such transmission mode measurements are being performed, the transmission mode may be considered as being active.

More generally, and regardless of whether the optical vital signs sensing device 10 is configured to perform measurements in the transmission mode only, such as in FIG. 2, or in both the reflection mode and the transmission mode, such as in FIGs 1 and 4, the device 10 may be controlled such that the transmission mode and/or the reflection mode may be selectively activated and deactivated.

In embodiments configured to perform vital signs measurements in both the transmission and the reflection mode, such as the devices 10 of FIGs. 1 and 4, each mode may be activated or deactivated individually or simultaneously, such that measurements may be performed in a single mode or both modes simultaneously.

Whilst the transmission mode may allow for a broader range of vital signs measurements to be obtained, e.g. heart rate, SpO2, breathing rate, the increased sensitivity of transmission mode measurements, e.g. the increased sensitivity to movement, may increase the likelihood of producing unreliable results. Accordingly, there is a risk that transmission measurements may be performed even when they are unlikely to yield reliable results.

Conversely, whilst it may be desirable to perform reflection measurements due to the higher likelihood of obtaining reliable measurements, e.g. due to the reduced movement sensitivity, the range of data available in the reflection mode may be more limited. Accordingly, valuable additional transmission mode data may be missed, when it could have in fact been reliably obtained.

Accordingly, it would be desirable to provide a simple way of controlling an optical vital signs sensing device 10 to increase the likelihood of performing reliable measurements.

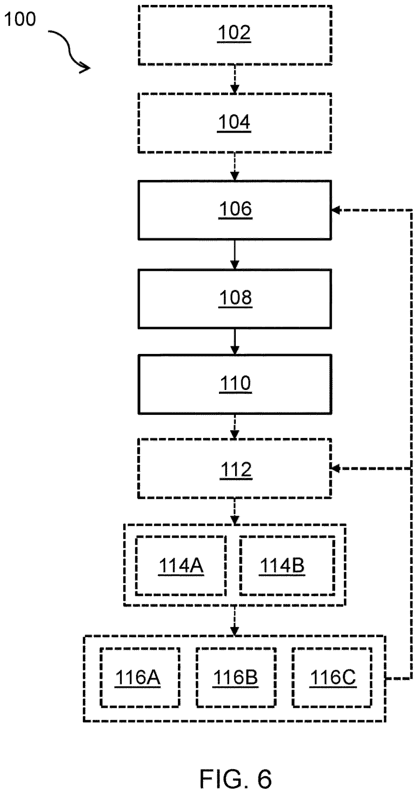

Referring to Fig. 6, the present disclosure accordingly provides a method 100 for controlling an optical vital signs sensing device 10.

The optical vital signs sensing device 10 may comprise a processor 28 configured to receive 106 at least one of motion data and reflection mode data, wherein the motion data is indicative of movement of an optical vital signs sensing device 10 and wherein the reflection mode data is associated with a reflection mode signal quality of a vital signs measurement performed by the optical vital signs sensing device 10 in a reflection mode. The processor 28 may be configured to determine 108 at least one of whether the movement satisfies a movement criterion by analyzing the motion data and whether the reflection mode signal quality satisfies a reflection mode minimum quality criterion by analyzing the reflection mode data. The processor 28 may be further configured to activate 110 the transmission mode of the optical vital signs sensing device 10 if at least one of the movement criterion and the reflection mode minimum quality criterion is satisfied.

By activating the transmission mode in this way, the likelihood of obtaining reliable vital signs measurements using the optical vital signs sensing device 10 may be increased. This is because the transmission mode will only be activated when data, e.g. motion data or reflection mode data, indicates that reliable transmission mode vital signs measurements may be obtained following activation of the transmission mode.

For example, if vital signs measurements performed in the reflection mode are good quality, e.g. the signal quality satisfies the minimum quality criterion, this may indicate a greater likelihood of obtaining reliable measurements in the transmission mode using the optical vital signs sensing device 10. Similarly, if movement of the device 10 is sufficiently low, e.g. the movement of the device 10 satisfies the movement criterion, this may indicate a greater likelihood of obtaining reliable measurements in the transmission mode, due to the greater sensitivity of transmission measurements to motion.

In such embodiments, the processor 28 may activate 110 the transmission mode by controlling operation of the optical emitter assembly 12 such that light is emitted from the optical emitter assembly 12 to the optical detector assembly 20, e.g. the first optical detector 22.

In some embodiments, the optical vital signs sensing device 10 further comprises a motion sensor 26, e.g. an accelerometer, for providing said motion data.

The motion data may comprise accelerometer data from the motion sensor 26, e.g. the accelerometer, indicative of acceleration of the optical vital signs sensing device 10. In such embodiments, the movement criteria may be satisfied when the motion data, e.g. the accelerometer data, is indicative of movement, e.g. acceleration, of the device 10 being below an acceleration threshold.

In a non-limiting example, whilst the device 10 is performing vital signs measurements on a body part in the reflection mode, the processor may receive 106 reflection mode data associated with a signal quality of a vital signs measurement performed by the device 10 in the reflection mode. If the processor 28 subsequently determines that the signal quality is sufficiently high, e.g. that the reflection mode signal quality satisfies the reflection mode minimum quality criterion, the processor 110 may activate the transmission mode such that light is transmitted from the optical emitter assembly 12 through the body part to reach the first optical detector 22. In such an example, the reflection mode may remain active or may be deactivated upon activation 110 of the transmission mode.

The reflection mode signal quality may comprise one or more of a signal strength and/or a signal to noise ratio of a vital signs measurement performed by the optical vital signs sensing device 10 in the reflection mode. That is, the reflection mode minimum quality criterion may be satisfied when the signal strength is above a minimum signal strength and/or when the signal to noise ratio is above a minimum signal to noise ratio.

In another non-limiting example, the processor 28 may receive motion data, e.g. motion data from motion sensor 26, indicative of movement of the device 10 whilst the reflection mode is active. If the processor 28 determines that movement is sufficiently low, e.g. that the movement satisfies the movement criterion, the processor 28 may activate 110 the transmission mode such that light is emitted from the emitter assembly 12 through the body part to reach the first optical detector 22. In such an example, the device 10 may be configured to perform vital signs measurements in both the transmission mode and the reflection mode, such as the device 10 of FIG. 1 and FIG. 4. Alternatively, the device 10 may be configured to perform measurements in the transmission mode only, e.g. there is no reflection mode, such as the device 10 of FIG. 2.

In any such examples, if neither the movement criterion nor the reflection mode minimum quality criterion are satisfied, the processor 28 may not activate the transmission mode.

In embodiments in which the optical vital signs sensing device 10 is controllable to perform vital signs measurements on a body part in a reflection mode, the motion data may be indicative of movement of the optical vital sign sensing device 10 whilst the reflection mode is active.

In some embodiments, the processor 28 may be configured to receive 106 both the motion data and the reflection mode data, and to determine 108 whether the movement satisfies the movement criterion by analyzing the motion data and whether the reflection mode signal quality satisfies the reflection mode minimum signal quality criterion by analyzing the reflection mode data. In such embodiments the processor 28 may further be configured to activate 110 the transmission mode subject to both the movement criterion and the reflection mode minimum quality criterion being satisfied.

In this way, the likelihood of obtaining reliable transmission mode vital signs measurements may be increased, compared to the situation when only one of the movement criterion or the reflection mode minimum signal quality criterion is satisfied.

In a non-limiting example, whilst the device 10 is performing vital signs measurements on a body part in the reflection mode, the processor may receive 106 reflection mode data associated with a signal quality of a vital signs measurement performed by the device 10 in the reflection mode and motion data indicative of movement of the device 10 whilst performing said reflection mode measurements. If the processor 28 subsequently determines that the signal quality is sufficiently high, e.g. that the reflection mode signal quality satisfies the reflection mode minimum quality criterion, and that that the movement is sufficiently low, e.g. that the movement satisfies the movement criterion, the processor 110 may activate the transmission mode such that light is transmitted from the optical emitter assembly 12 through the body part to reach the first optical detector 22. In such an example, the reflection mode may remain active or may be deactivated upon activation 110 of the transmission mode.

In some embodiments, the processor 28 may be configured to receive 112 transmission mode data, wherein the transmission mode data is associated with a transmission mode signal quality of a vital signs measurement performed by the optical vital signs sensing device 10 in the transmission mode.

That is, the transmission mode data may be received following activation of the transmission mode.

In this way, the optical vital signs sensing device 10 may be further controlled whilst the transmission mode is active.

In embodiments in which the optical vital signs sensing is a dual-mode device, such as the devices 10 of FIGs. 1 and 4, the transmission mode data may be received whilst both the reflection mode and the transmission mode are active, or whilst the transmission mode is active, and the reflection mode is inactive.

In some embodiments, the processor 28 is configured to determine 114A whether the transmission mode signal quality satisfies a transmission mode minimum quality criterion by analyzing the transmission mode data, and deactivating the reflection mode if the transmission mode minimum quality criterion is satisfied.

In this way, the likelihood of obtaining reliable measurements may be increased. This is because the reflection mode may not be deactivated until reliable measurements have been obtained in the transmission mode. This may also ensure continuity of measurement.

In a non-limiting example, once the transmission mode has been activated 110, the processor 28 may receive 112 transmission mode data. Subsequently, the processor 28 may determine 114A whether the transmission mode signal quality satisfies the transmission mode minimum quality criterion. If the quality criterion is satisfied, e.g. the transmission mode signal quality is sufficiently high, the processor 28 may deactivate the reflection mode such that only the transmission mode remains active.

The transmission mode signal quality may comprise one or more of a signal strength and/or a signal to noise ratio of a vital signs measurement performed by the optical vital signs sensing device 10 in the transmission mode. That is, the transmission mode minimum quality criterion may be satisfied when the signal strength is above a minimum signal strength and/or when the signal to noise ratio is above a minimum signal to noise ratio.

In some embodiments, the processor 28 is configured to determine 114B whether movement of the optical vital signs sensing device 10 while the transmission mode is active satisfies a further movement criterion by analyzing transmission mode motion data, and wherein the method further comprises at least one of deactivating 116B the transmission mode if the further movement criterion is satisfied and activating 116C the reflection mode if the further movement criterion is satisfied.

In this way, the likelihood of obtaining reliable measurements may be increased, whilst also ensuring continuity of measurement.

The transmission mode motion data may comprise transmission mode accelerometer data indicative of acceleration of the optical vital signs sensing device 10 whilst the transmission mode is active. In such embodiments, the further movement criterion may be satisfied when the transmission mode motion data, e.g. the transmission mode accelerometer data, is indicative of movement, e.g. acceleration, of the device 10 being above a transmission mode acceleration threshold.

The transmission mode motion data may be obtained from the motion sensor 26.

In a non-limiting example, if the processor 28 determines that the further movement criterion is satisfied, e.g. that movement of the device 10 is high, the processor 28 may deactivate 116B the transmission mode as it is less likely that reliable results will be obtained due to the high movement of the device 10.

In a non-limiting example, if the processor 28 determines that the movement criterion is satisfied, e.g. that movement of the device 10 is high, the processor 28 may activate 116C the reflection mode to increase the likelihood of obtained reliable vital signs measurements due to the reduced sensitivity of the reflection mode to movement. In such examples, the transmission mode may remain active, or the processor may deactivate 116B the transmission mode such that only the reflection mode remains active.

In some embodiments, the processor 28 is configured to determine 102 whether movement of the optical vital signs sensing device 10 while no transmission mode and no reflection mode are active satisfies an initial movement criterion by analyzing initial motion data and activating 104 at least one of the reflection mode and the transmission mode if the initial movement criterion is satisfied.

In this way, the vital signs sensing device 10 may be further controlled whilst the device is not being used to perform vital signs measurements, i.e. whilst the device 10 is in a standby mode, prior to performing a measurement.

The initial motion data may comprise initial accelerometer data indicative of acceleration of the optical vital signs sensing device 10 while no transmission mode and no reflection mode are active. In such embodiments, the initial movement criterion may be satisfied when the initial motion data, e.g. the initial accelerometer data, is indicative of movement, e.g. acceleration, of the device 10 being above an initial acceleration threshold.

In some embodiments, the device 10 may comprise a user interface (not shown). For example, the user interface may be comprised by the optical vital signs sensing device 10 itself or may be comprised by an external device such as a smartphone.

In some embodiments, the processor 28 is configured to generate a signal for creating a notification that the vital signs sensing device is being used incorrectly.

In this way, a user of the device 10 may be alerted when the device 10 is being used incorrectly so that they can rectify this, increasing the likelihood of obtaining reliable vital signs measurements.

For example, the notification may be issued via the user interface.

In some embodiments, the processor 28 is further configured to receive environmental light data of an environment in which the vital signs sensing device 10 is being used, and wherein the generating the signal for creating the notification is based at least partly on the environmental light data.

Environmental light levels may be indicative of incorrect use, e.g. incorrect positioning, of the optical vital signs sensing device 10.

For example, environmental light levels may be indicative of the device 10 being incorrectly positioned on the skin of the user such that the likelihood of performing reliable vital signs measurements is reduced.

In such embodiments, the environmental light data may comprise a light intensity as measured by the optical detector assembly 20, e.g. the first optical detector 22 or the second optical detector 24. For example, if the light intensity is low, this may indicate that the device 10 is positioned on the skin. If the light intensity is high, this may indicate that the device 10 is not correctly positioned on the skin.

In a non-limiting illustrative example, the device 10 may have become dislodged whilst performing vital signs measurements on a user such that the optical detector assembly 20 is exposed to high environmental light intensity, e.g. high background light levels, resulting in a reduction in measurement signal quality. In this scenario, the signal may be generated, and the notification subsequently issued to a user, e.g. issued via the user interface.

In some embodiments, the processor 28 may be configured to generate the signal for creating the notification based at least partly on the reflection mode data.

Reflection mode data may be indicative of incorrect use of the optical vital signs sensing device 10. In this way, the likelihood of obtaining reliable optical vital signs measurements may be increased.

For example, low quality vital signs measurements performed in the reflection mode may be indicative of incorrect use of the optical vital signs sensing device 10.

In a non-limiting illustrative example, if environmental light levels at the optical detector assembly 20 low, indicating that the sensor has been placed on the skin, whilst reflection mode signal quality is low, this may indicate that the user needs to reposition the device 10. Accordingly, the processor 28 may generate the signal for creating the notification.

Alternatively or additionally, the generating the signal for creating the notification may be based on the motion data.

Alternatively or additionally, the generating the signal for creating the notification may be based on the transmission mode motion data and/or the transmission mode data.

In some embodiments the reflection mode data and/or the transmission mode data is indicative of one or more heart rate measurements, blood oxygen measurements and/or breathing measurements.

In a non-limiting example, the reflection mode data may comprise one or more heart rate measurements.

In a non-limiting example, the transmission mode data may comprise one or more heart rate measurements, blood oxygen measurements and/or breathing measurements.

In a non-limiting example, green light is emitted by the emitter assembly 12 whilst the reflection mode is active, and at least one of red and infrared light is emitted by the emitter assembly 12 whilst the transmission mode is active.

Operation of the optical vital signs sensing device 10 according to the method 100 will be described with reference to the non-illustrative example of FIG. 5, which provides a flow diagram 300 of a typical use case of an optical vital signs sensing device 10, in which an optical vital signs sensing device 10 is used to monitor vital signs of a baby.

In this non-limiting example 300, the optical vital signs sensing device 10 is configured to be placed on the baby's foot. However, it is noted that the operating principles of the optical vital signs sensing device 10 described in this example 300 are equally applicable to monitoring of any body part, and to the monitoring of adults and children of any age. For example, the principles may be equally applicable to a smart watch 10 placed on the wrist of an adult.

At block 302 the optical vital signs sensing device 10 is picked up and placed on the baby's foot, and the baby is put to bed. For example, the optical vital signs sensing device 10 may be picked up from a docking station in which the device 10 was stationary and no transmission mode and no reflection mode are active.

Upon picking up the device 10 and placing the device 10 on the baby's foot at block 302, the processor 28 may determine 102 whether movement of the device 10 satisfies the initial movement criterion by analyzing initial motion data, such as initial motion data from motion sensor 26. Accordingly, if the initial movement criterion is satisfied at block 302, the processor 28 may automatically activate 104 the reflection mode.

For a period of time immediately following putting the baby to bed at block 302, it may be that the baby is restless, e.g. the baby is moving. In this situation, the device 10, such as the device 10 of FIG. 1, may perform measurements in the reflection mode only, whilst the transmission mode remains inactive, due to the reduced sensitivity of the reflection mode to movement.

That is, the processor 28 may receive 106 motion data indicative of the baby's movement. If the processor 28 determines 108 that the movement does not satisfy the movement criterion, e.g. because the baby is restless and movement of the device 10 is high, the processor 28 may continue to operate in the reflection mode, e.g. the transmission mode will not be activated 110.

In such an example, the processor 28 may continually receive 106 motion data such that the processor 28 may activate 110 the transmission mode at a later time, e.g. if the baby is still and the movement criterion is satisfied.

At block 302, if no reliable reflection mode measurements can be obtained, whilst environmental light levels are low, e.g. light levels at the optical detector assembly 20 are low indicating that the device is positioned on the skin, the processor 28 may generate a signal for creating a notification that the vital signs sensing device is being used incorrectly, e.g. the device 10 is placed incorrectly. That is, the processor 28 may generate the signal based on received environmental light data and the reflection mode data.

At block 304 the baby falls asleep, and subsequently falls into a deep sleep.

At this point, the device 10 may still be operating in the reflection mode only. Whilst the reflection mode is active, and as described above, the processor 28 may continue to receive 106 motion data and reflection mode data. In this way, it may be continually assessed, e.g. at regular intervals, whether the transmission mode is likely to yield reliable results.

Once the baby has fallen asleep at block 304, the baby's movement level may be lower, e.g. low enough that reliable transmission mode measurements can be performed. Accordingly, if the processor 28 determines that the movement criterion is satisfied, the processor 28 may activate 110 the transmission mode, and the device 10 may begin performing vital signs measurements in the transmission mode.

Initially, upon activating the transmission mode, the reflection mode may remain active such that vital signs measurements may still be performed in the reflection mode. In this way, if reliable transmission modes are unable to be obtained, measurements may still be obtained in the reflection mode ensuring continuity of measurement.

If, following activation 110 of the transmission mode at block 304, reliable transmission mode vital signs measurements are obtained, the reflection mode may be deactivated 116A. That is, the processor 28 may receive 112 transmission mode data, determine 114A whether the transmission mode signal quality satisfies the transmission mode minimum signal quality criterion, and deactivate 116A the reflection mode if the transmission mode minimum quality criterion is satisfied. Alternatively, the device 10 may continue to perform measurements in both the transmission mode and the reflection mode simultaneously.

At block 304, if reliable transmission mode measurements cannot be obtained whilst reflection mode measurements were of good quality, the processor 28 may generate a signal for creating a notification that the vital signs sensing device 10 is being used incorrectly based on the reflection mode data. For example, the device 10 may be positioned incorrectly on the skin.

At block 306, the baby wakes up.

For example, once the baby has woken up, the movement of the device 10 and the baby may be higher, e.g. higher than when the baby was asleep at block 304.

The processor 28 may now determine 114B whether movement of the optical vital signs sensing device 10 satisfies a further movement criterion by analyzing transmission motion data.

Accordingly, if movement is high such that the further movement criterion is satisfied, the processor 28 may deactivate 116B the transmission mode, since the likelihood of obtaining reliable transmission mode measurements may be lower. If prior to deactivating 116B the transmission mode both the transmission mode and the reflection mode were active, the reflection mode may remain active following deactivation 116B of the transmission mode.

Alternatively, if prior to deactivating 116B the transmission mode only the transmission mode was active, the processor may activate 116C the reflection mode if the processor 28 has determined that the that the further movement criterion has been satisfied.

Alternatively, upon determining that the further movement criterion has been satisfied, the processor 28 may not deactivate 116B the transmission mode and may instead activate 116C the reflection mode such that both the transmission mode and the reflection mode are active simultaneously.

The optical vital signs sensing device 10 may subsequently be removed, and the monitoring session may be ended.

It is noted that FIG. 6 provides a flowchart of the method 100 for controlling an optical vital signs sensing device 10 described above with reference to FIGs. 1, 2 and 4.

The method 100 comprises receiving 106 at least one of motion data and reflection mode data, wherein the motion data is indicative of movement of an optical vital signs sensing device 10 and wherein the reflection mode data is associated with a reflection mode signal quality of a vital signs measurement performed by the optical vital signs sensing device 10 in a reflection mode; determining 108 at least one of: whether the movement satisfies a movement criterion by analyzing the motion data; and whether the reflection mode signal quality satisfies a reflection mode minimum quality criterion by analyzing the reflection mode data; and activating 110 a transmission mode of the optical vital signs sensing device 10 if at least one of the movement criterion and the reflection mode minimum quality criterion is satisfied.

The method 100 may be performed using the processor 28 of the optical vital signs sensing device 10 described above.

Further provided is a computer program comprising computer-readable instructions encoding the steps of the method 100 of any of the embodiments disclosed herein. For example, when the computer program is run on a processor 28, the computer program product may cause the processor 28 to implement the method 100 according to any of the embodiments discussed herein.

Also provided is a processor configured to execute the computer program.

Further provided is a computer program comprising computer-readable instructions encoding the steps of the method 100 of any embodiments described herein. For example, when the computer program is run on a processor 28, the computer program product may cause the processor 28 to implement the method 100 according to any of the embodiments discussed herein.

As discussed above, embodiments make use of a processor 28. The processor 28 can be implemented in numerous ways, with software and/or hardware, to perform the various functions required. The processor 28 may, for example, employ one or more microprocessors that may be programmed using software (e.g., microcode) to perform the required functions. The processor 28 may however be implemented with or without employing a microprocessor, and also may be implemented as a combination of dedicated hardware to perform some functions and microprocessor(s) (e.g. one or more programmed microprocessors and associated circuitry) to perform other functions.

Examples of processor 28 components that may be employed in various embodiments of the present disclosure include, but are not limited to, conventional microprocessors, application specific integrated circuits (ASICs), and field-programmable gate arrays (FPGAs).

In various implementations, a processor or processor 28 may be associated with one or more storage media such as volatile and non-volatile computer memory such as RAM, PROM, EPROM, and EEPROM. The storage media may be encoded with one or more programs that, when executed on one or more processors and/or processors 28, perform the required functions. Various storage media may be fixed within a processor or processor 28 or may be transportable, such that the one or more programs stored thereon can be loaded into a processor or processor 28.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor 28 or processor may be implemented by a single processor or by multiple separate processing units which may together be considered to constitute a "processor" or "processor". Such processing units may in some cases be remote from each other and communicate with each other in a wired or wireless manner.

Still further provided a method comprising causing, using a first processor, a second processor, e.g. the processor 28, to execute a computer program comprising computer-readable instructions encoding the steps of the method 100 of any of the embodiments disclosed herein. The first processor may be separate from the second processor 28 that are each a processing system, e.g. a distributed system in which the first processor is remote from the second processor.

Yet further provided is a computer program comprising computer-readable instructions encoding the step of causing, using a first processor, a second processor, e.g. the processor 28, to execute a computer program comprising computer-readable instructions encoding the steps of the method 100 of any of the embodiments disclosed herein. Further provided is provided a processor, e.g. the first processor, configured to execute said computer program.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method (100) comprising:
receiving (106) at least one of motion data and reflection mode data, wherein the motion data is indicative of movement of an optical vital signs sensing device and wherein the reflection mode data is associated with a reflection mode signal quality of a vital signs measurement performed by the optical vital signs sensing device in a reflection mode;
determining (108) at least one of:
whether the movement satisfies a movement criterion by analyzing the motion data; and
whether the reflection mode signal quality satisfies a reflection mode minimum quality criterion by analyzing the reflection mode data; and
activating (110) a transmission mode of the optical vital signs sensing device if at least one of the movement criterion and the reflection mode minimum quality criterion is satisfied.

2. The method (100) according to claim 1, wherein the receiving (106) comprises receiving the motion data and the reflection mode data, and the determining (108) comprises determining whether the movement satisfies the movement criterion and whether the reflection mode signal quality satisfies the reflection mode minimum signal criterion, and wherein the activating (110) the transmission mode is subject to the movement criterion and the reflection mode minimum quality criterion being satisfied.

3. The method (100) according to claim 1 or 2, further comprising receiving (112) transmission mode data, wherein the transmission mode data is associated with a transmission mode signal quality of a vital signs measurement performed by the optical vital signs sensing device in the transmission mode.

4. The method (100) according to claim 3 further comprising:
determining (114A) whether the transmission mode signal quality satisfies a transmission mode minimum quality criterion by analyzing the transmission mode data; and
deactivating (116A) the reflection mode if the transmission mode minimum quality criterion is satisfied.

5. The method (100) according to any one of claims 1 to 4, further comprising:
determining (114B) whether movement of the optical vital signs sensing device while the transmission mode is active satisfies a further movement criterion by analyzing transmission mode motion data, and wherein the method (100) further comprises at least one of:
deactivating (116B) the transmission mode if the further movement criterion is satisfied; and
activating (116C) the reflection mode if the further movement criterion is satisfied.

6. The method (100) according to any of claims 1 to 5, further comprising:
determining (102) whether movement of the optical vital signs sensing device while no transmission mode and no reflection mode are active satisfies an initial movement criterion by analyzing initial motion data; and
activating (104) at least one of the reflection mode and the transmission mode if the initial movement criterion is satisfied.

7. The method (100) according to any of claims 1 to 6, further comprising generating a signal for creating a notification that the vital signs sensing device is being used incorrectly.

8. The method according to claim 7, further comprising receiving environmental light data of an environment in which the vital signs sensing device is being used, and wherein the generating the signal for creating the notification is based at least partly on the environmental light data.

9. A computer program comprising computer-readable instructions encoding the steps of the method of any of claims 1 to 8.

10. A processor (28) configured to execute the computer program of claim 9 to carry out the method of any of claims 1 to 8.

11. An optical vital signs sensing device (10) comprising:
an optical emitter assembly (12); and
an optical detector assembly (20) comprising a first optical detector (22);
the optical vital signs sensing device (10) being configured to perform vital signs measurements, when the device is arranged on a body part, in at least a transmission mode in which light from the optical emitter assembly (12) is transmitted through the body part to reach the first optical detector (22), **characterized in that** the optical vital signs sensing device (10) comprises the processor (28) of claim 10.

12. The optical vital signs sensing device (10) of claim 11, wherein the optical detector assembly (20) comprises a second optical detector (24) and wherein the optical vital signs sensing device (10) is configured to perform vital signs measurements on the body part in the reflection mode in which light from the optical emitter assembly (12) is emitted into and reflected from the body part to the second optical detector (24).

13. A method comprising using a first processor to cause a second processor to execute a computer program comprising computer-readable instructions encoding the steps of the method of any of claims 1 to 8.

14. A computer program comprising computer-readable instructions encoding the step of the method of claim 13.

15. A processor configured to execute the computer program of claim 14 to carry out the method of claim 13.
